# EUROPEAN PATENT APPLICATION

(11) **EP 4 772 237 A1**
(43) Date of publication of application: **08.07.2026**
(21) Application number: 24860456.3
(22) Date of filing: 30.08.2024
(51) Int. Cl.: A61N 5/10, A61B 6/03, A61B 5/113, G06N 3/08, G16H 20/40

(54) **METHOD AND APPARATUS FOR OPTIMIZING BEAM ANGLE FOR PROTON THERAPY**

(30) Priority: 30.08.2023 KR 20230114403
(71) Applicant: Samsung Life Public Welfare Foundation, Seoul 04348 (KR)
(72) Inventor: AHN, Sang Hee, Seoul 06351 (KR); PARK, Hee Chul, Seoul 06351 (KR); YU, Jeong II, Seoul 06351 (KR)
(74) Representative: Ostertag & Partner Patentanwälte mbB
(86) International application number: PCT/KR2024/013044
(87) International publication number: WO 2025/048538

(57) **Abstract**

A beam angle optimization method for proton therapy comprises: receiving, by an analysis apparatus, distance information from a patient's body surface to a tumor, internal heterogeneity information, internal transmission information of a proton beam, and internal motion information according to the patient's respiration; inputting, by the analysis apparatus, the distance information from the patient's body surface to the tumor, the internal heterogeneity information, the internal transmission information of the proton beam, and the internal motion information according to the patient's respiration into a deep learning model; and calculating, by the analysis apparatus, a proton therapy beam angle based on an output value of the deep learning model.

## Description

### [Technical Field]

The present disclosure relates to a method and an apparatus for optimizing a proton beam angle during proton therapy by utilizing deep learning technology.

### [Background Art]

Radiation therapy is a treatment that destroys tumors by using radiation such as X-rays, gamma rays, electron beams, or proton beams. Among radiation therapies, proton therapy is a treatment method that destroys cancer tissues by using protons. proton therapy is a treatment method based on the Bragg Peak principle, which can destroy a tumor while minimizing damage to normal tissues by using protons. The Bragg Peak principle refers to a phenomenon in which a proton beam emits an enormous amount of radiation energy at the moment when it passes through normal tissue and reaches the tumor.

### [Prior Art Document]

### [Patent Document]

U.S. Patent Application Publication No. 2021/0101027

### [DISCLOSURE]

### [Technical Problem]

In proton therapy, due to the characteristics of the Bragg Peak, changes in internal organs can greatly affect the treatment. Therefore, before performing proton therapy, it is necessary to establish an optimal treatment plan so that the therapeutic radiation can be delivered to the tumor as planned while considering organ movement. Specifically, when performing proton therapy, a treatment plan must be established to minimize damage to critical organs around the tumor caused by radiation while allowing the maximum amount of radiation to be delivered to the tumor region.

In particular, determining the angle of a proton beam when establishing a treatment plan is very important in proton therapy. For example, when a proton beam passes through a path containing many heterogeneous materials, its range can change, increasing the likelihood that the beam will not be delivered as intended. This also holds when an organ subject to respiratory motion lies along the beam path. In such a case, the proton beam may be delivered to a normal organ instead of the tumor or may fail to deliver a sufficient dose to the tumor, which may cause the recurrence of cancer in the treated area.

However, determining the proton beam angle requires highly advanced clinical experience and knowledge, and there has been a problem in that it takes a long time to determine the beam angle.

The present disclosure aims to provide a method that optimizes the proton beam angle during proton therapy by utilizing deep learning technology.

### [Technical Solution]

A beam angle optimization method for proton therapy, comprising: receiving, by an analysis apparatus, distance information from a patient's body surface to a tumor, internal heterogeneity information, internal transmission information of a proton beam, and internal motion information according to the patient's respiration; inputting, by the analysis apparatus, the distance information from the patient's body surface to the tumor, the internal heterogeneity information, the internal transmission information of the proton beam, and the internal motion information according to the patient's respiration into a deep learning model; and calculating, by the analysis apparatus, a proton therapy beam angle based on an output value of the deep learning model.

### [Advantageous Effects]

By using the technology described below, the time consumed in establishing a proton therapy plan can be reduced while improving the quality of the proton therapy plan. Through this process, it is possible to reduce the waiting time for patients receiving proton therapy or to increase the number of patients who can receive proton therapy.

### [Brief Description of Drawings]

FIG. 1 illustrates an overall process in which an analysis apparatus (100) optimizes a proton beam angle.
FIG. 2 shows one embodiment (200) of a proton beam angle optimization method.
FIG. 3 illustrates one embodiment of distance information from a patient's body surface to a tumor and internal heterogeneity information.
FIG. 4 shows one embodiment of internal motion information.
FIG. 5 illustrates one embodiment of a process of a patient-specific beam angle calculation technique for proton therapy.
FIG. 6 shows one embodiment for calculating a therapeutic effect according to a proton beam treatment angle.
FIG. 7 shows another embodiment in which an analysis apparatus calculates a proton therapy beam angle.
FIG. 8 shows one embodiment of the configuration of an analysis apparatus (300).

### [Mode for Disclosure]

The technology described below can be modified in various ways and may have various embodiments. Specific embodiments of the technology described below may be described in the drawings of the specification. However, this is for describing the technology described below, and is not intended to limit the technology described below to specific embodiments. Accordingly, it should be understood that all modifications, equivalents, or alternatives included in the spirit and technical scope of the technology described below are included in the technology described below.

In the terms used below, unless clearly interpreted otherwise in context, singular expressions should be understood to include plural expressions, and terms such as "comprise" should be understood as indicating the presence of listed features, numbers, steps, operations, components, parts, or combinations thereof, rather than excluding the presence or addition of one or more other features, numbers, steps, operations, components, parts, or combinations thereof.

Before describing the drawings in detail, it is intended to clarify that the classification of components in this specification is merely a classification according to the main functions performed by each component. That is, two or more components to be described below may be combined into one component, or one component may be divided into two or more components according to more segmented functions and provided. In addition, each of the components to be described below may additionally perform some or all of the functions among the functions performed by other components, and some of the functions among the main functions performed by each component may be performed exclusively by another component.

In performing a method or an operation method, unless a specific order is clearly described in context for each process constituting the method, the processes may occur in an order different from the stated order. That is, the processes may occur in the same order as stated, may be performed substantially simultaneously, or may be performed in the reverse order.

The technology described below relates to a patient-specific beam angle calculation technique for proton therapy.

Hereinafter, it is described that an analysis apparatus optimizes a beam angle by using a learning model. The analysis apparatus may be implemented as various devices capable of data processing. For example, the analysis apparatus may be implemented as a PC, a server on a network, a smart device, or a chipset in which a dedicated program is embedded.

Hereinafter, an overall process in which the analysis apparatus performs a method of optimizing a proton beam angle is described.

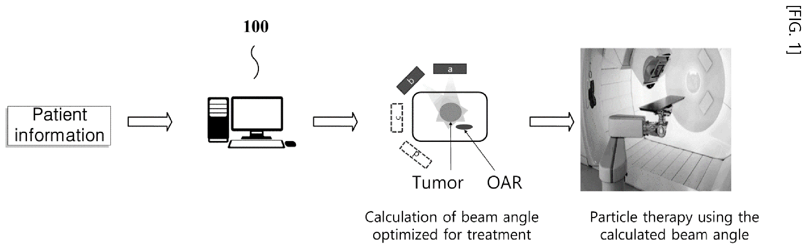

FIG. 1 illustrates an overall process in which an analysis apparatus (100) optimizes a beam angle for proton therapy.

The analysis apparatus may receive distance information from a patient's body surface to a tumor, internal heterogeneity information, internal transmission information of a proton beam, and internal motion information according to the patient's respiration. The analysis apparatus may input the distance information to the tumor, the internal heterogeneity information, the internal transmission information of the proton beam, and the internal motion information according to the patient's respiration into a deep learning model. The analysis apparatus may calculate a proton therapy beam angle based on an output value of the deep learning model. proton therapy may be performed based on the proton therapy beam angle thus calculated.

Hereinafter, a method of optimizing a proton beam angle is described in detail.

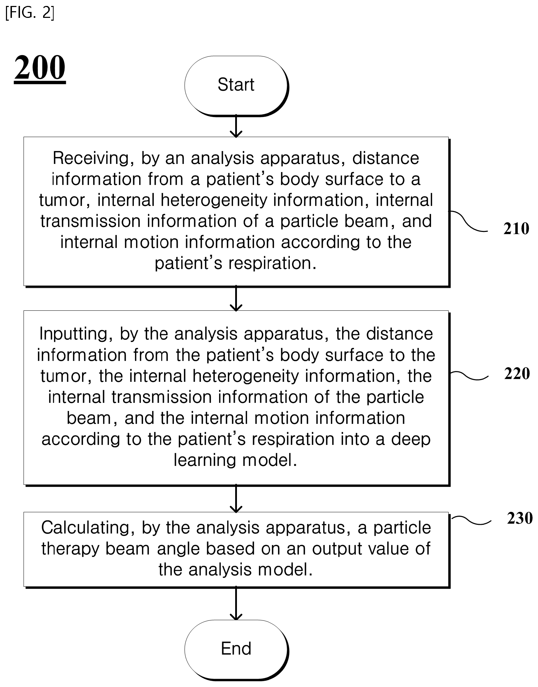

FIG. 2 is one embodiment (200) of a proton beam angle optimization method.

The analysis apparatus may receive distance information from a patient's body surface to a tumor, internal heterogeneity information, internal transmission information of a proton beam, and internal motion information according to the patient's respiration (210).

The distance information from the patient's body surface to the tumor may mean a distance (radiological depth) through which the proton beam passes from the patient surface to the tumor. That is, the distance information from the patient's body surface to the tumor may mean how far the tumor is from the patient's surface.

The internal heterogeneity information may include information on how homogeneous the interior of the body through which the proton beam passes is. Specifically, the internal heterogeneity information may be information calculated based on a standard deviation value of HU (Hounsfield Unit) acquired from a CT image.

The internal transmission information of the proton beam may mean how well the proton beam passes through the body. Specifically, the internal transmission information of the proton beam may mean information on how much a critical organ in the body transmits the proton beam. For example, the internal transmission information of the proton beam may include a penetration weight of OAR (Organs at Risk) indicating a degree to which the proton beam passes through a critical organ in the body.

The internal motion information according to the patient's respiration may mean information on a degree to which internal organs change as the patient breathes. Specifically, the internal motion information according to the patient's respiration may include information calculated from DVF (deformation vector fields). This may be acquired from 4D CT containing the patient's respiration information. In one embodiment, the internal motion information may include DVF values calculated from 4D CT at breathing phases 0 to 90. Alternatively, the internal motion information may include DVF values calculated from 4D CT at breathing phases 0 to 50. Alternatively, the internal motion information may include DVF values calculated from 4D CT at breathing phases 50 to 90.

The distance information from the patient's body surface to the tumor, the internal heterogeneity information, the internal transmission information of the proton beam, and the internal motion information according to the patient's respiration may be information acquired from a CT (Computed Tomography) image. Specifically, the information may be acquired from 4D CT images reflecting the patient's respiratory motion.

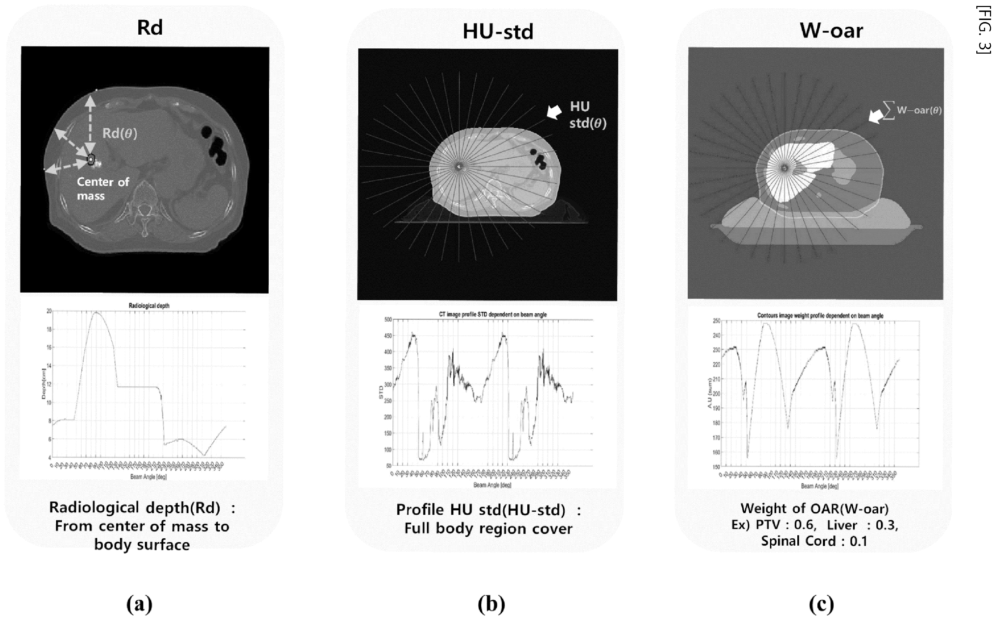

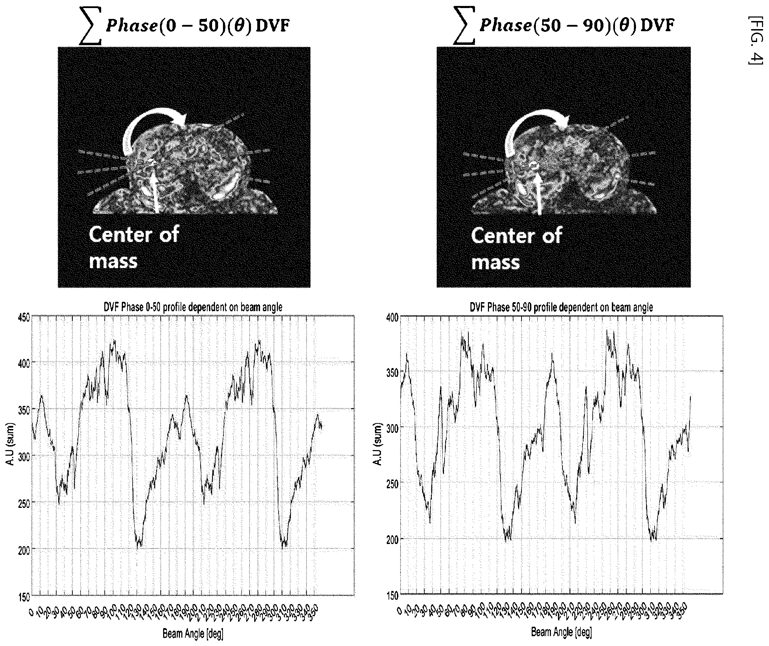

The distance information from the patient's body surface to the tumor, the internal heterogeneity information, the internal transmission information of the proton beam, and the internal motion information according to the patient's respiration may be information according to a proton beam angle. For example, as shown in FIGS. 3 and 4, the distance information from the patient's body surface to the tumor, the internal heterogeneity information, the internal transmission information of the proton beam, and the internal motion information according to the patient's respiration may have a form of graphs. FIG. 3(a) shows the distance information from the body surface to the tumor. FIG. 3(b) shows the internal heterogeneity information. FIG. 3(c) shows the internal transmission information of the proton beam. FIG. 4 shows the internal motion information according to respiration.

The analysis apparatus may input the distance information from the patient's body surface to the tumor, the internal heterogeneity information, the internal transmission information of the proton beam, and the internal motion information according to the patient's respiration into a deep learning model (220).

The deep learning model may be a model trained to output a proton therapy beam angle based on training data.

In one embodiment, the deep learning model may include a model based on an artificial neural network. For example, the deep learning model may include CNN (Convolutional Neural Network), RNN (Recurrent Neural Network), RBM (Restricted Boltzmann Machine), DBN (Deep Belief Network), and GAN (Generative Adversarial Network), RL (Relation Networks), and a Transformer Neural Network.

The analysis apparatus may calculate a proton therapy beam angle based on an output value of the deep learning model (230).

In one embodiment, the analysis apparatus may calculate a proton therapy beam angle that is determined to be most suitable for performing proton therapy among 360 degrees. For example, the analysis apparatus may derive, in the form of a peak or a specific region of a predicted graph, a portion that is evaluated to have the best therapeutic effect among 360 degrees.

The following describes one embodiment for constructing a deep learning model.

First, data of patients who have received proton therapy may be collected. The patient data may include 4D CT images containing the patient's respiratory information.

The data of patients who have received proton therapy may be processed.

In one embodiment, heterogeneous region information of patient tissue may be extracted from CT images. The heterogeneous region information may include Air, Bone, Metal material (Gold fiducial, stent), and Tissue, etc. Specifically, it may be possible to distinguish regions where organs exist or regions where tumors exist from CT images. For this purpose, a segmentation model may be used. The segmentation model may be a trained learning model trained to segment heterogeneous regions in CT images or to distinguish regions where tumors exist. For example, the segmentation model may be a model based on a U-net or a Transformer.

Alternatively, in one embodiment, based on the CT image and the information of organ delineation, the weight of a path through which a proton beam passes in the tumor and normal tissue may be adjusted.

Alternatively, in one embodiment, after setting a center of mass position in the tumor information, the distance from the surface to the center of mass position may be calculated to compute the distance information from the surface to the tumor.

The deep learning model may be trained using the processed patient data.

The training may be performed after converting the processed patient data into one-dimensional data.

After the training is completed, the derived beam angle may be compared with the actual treatment result to evaluate the model.

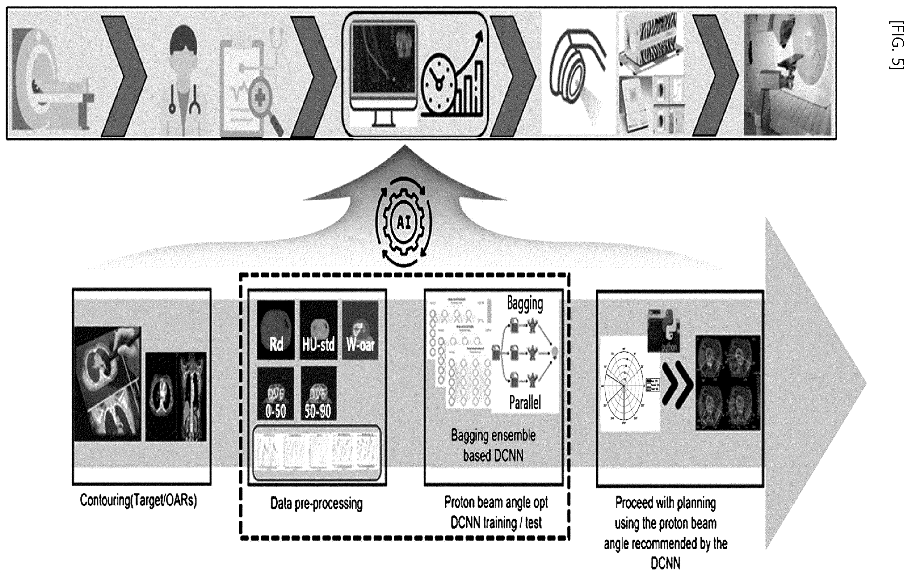

FIG. 5 illustrates one embodiment of a process of a patient-specific beam angle calculation technique for proton therapy. As shown in FIG. 5, the patient-specific beam angle calculation technique for proton therapy may include contouring medical images, performing data processing based on the contouring result, and then training or testing a deep learning model such as a DCNN (Deep Convolutional Neural Network). Subsequently, based on the deep learning model such as the DCNN, a patient-specific beam angle for proton therapy may be determined.

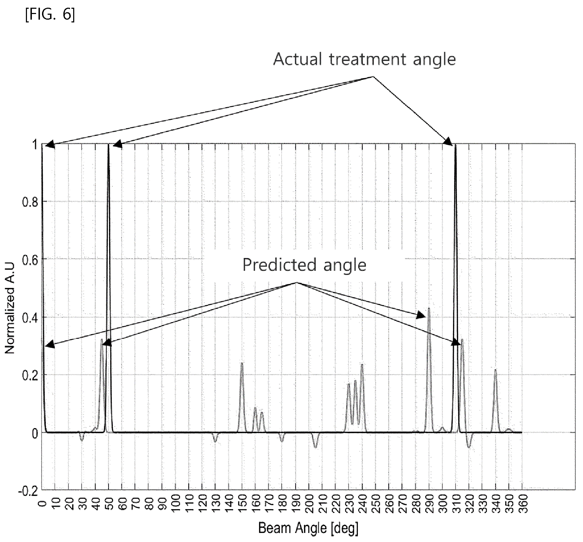

FIG. 6 shows one embodiment in which an analysis apparatus calculates a proton therapy beam angle. The analysis apparatus calculates an effect according to an angle and displays it in a graph.

**It** can be confirmed that, among the predicted results by the analysis apparatus, the result having a high peak substantially coincides with an actual treatment planning angle (reference data). In other words, it can be confirmed that the experimental proton therapy beam angle predicted by the analysis apparatus is reliable.

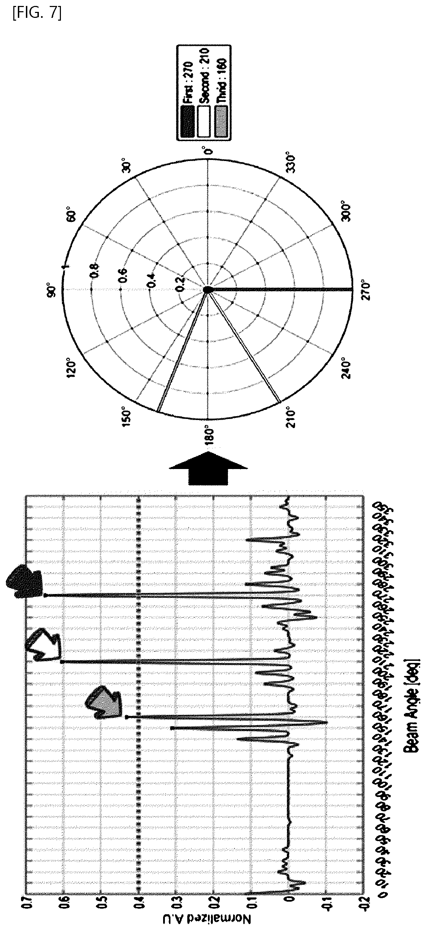

FIG. 7 shows another embodiment in which the analysis apparatus calculates a proton therapy beam angle. The analysis apparatus calculates an effect according to the beam angle and displays it as a graph. From the effect according to the predicted beam angle, the analysis apparatus may select a beam angle having an effect above a preset threshold. As shown in FIG. 7, three optimal beam angles (First, Second, and Third) may be selected. proton therapy may be performed based on the beam angles thus selected.

Hereinafter, the analysis apparatus will be described.

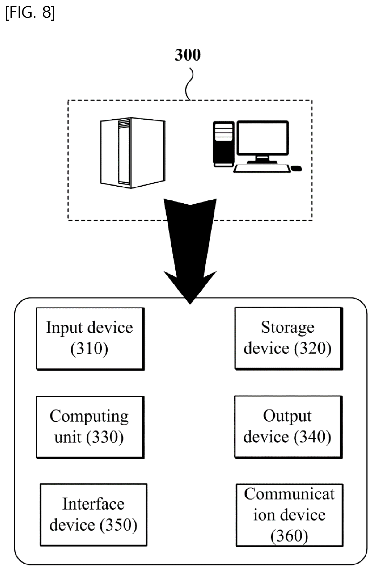

FIG. 8 shows one embodiment of a configuration of an analysis apparatus (300).

The analysis apparatus (300) may correspond to the analysis apparatus (100) described in FIG. 1. The analysis apparatus (300) may be a device that performs the aforementioned proton beam optimization method.

The analysis apparatus (300) may be physically implemented in various forms. For example, the analysis apparatus (300) may take the form of a PC, a notebook, a smart device, a server, or a data-processing dedicated chipset.

The analysis apparatus (300) may include an input device (310), a storage device (320), a computing unit (330), an output device (340), an interface device (350), and a communication device (360).

The input device (310) may include an interface device (such as a keyboard, mouse, or touchscreen) for receiving certain commands or data. The input device (310) may include a configuration for receiving information through a separate storage device (such as USB, CD, or hard disk). The input device (310) may receive data through a separate measuring device or from a separate database. The input device (310) may receive data through wired or wireless communication.

The input device (310) may receive information and a model necessary for performing the aforementioned proton beam optimization method. The input device (310) may receive distance information from a patient's body surface to a tumor, internal heterogeneity information, internal transmission information of a proton beam, and internal motion information according to the patient's respiration. The input device (310) may receive a deep learning model.

The storage device (320) may store information received through the input device (310). The storage device (320) may store information generated during a computation process performed by the computing unit (330). That is, the storage device (320) may include a memory. The storage device (320) may store results calculated by the computing unit (330).

The storage device (320) may store information and a model necessary for performing the aforementioned proton beam optimization method. The storage device (320) may store distance information from a patient's body surface to a tumor, internal heterogeneity information, internal transmission information of a proton beam, and internal motion information according to the patient's respiration.

The computing unit (330) may be a device such as a processor, an application processor (AP), or a chip in which a program is embedded, that processes data and performs certain computations. The computing unit (330) may generate a control signal that controls the analysis apparatus (300).

The computing unit (330) may perform computations necessary for performing the aforementioned proton beam optimization method. The computing unit (330) may input the distance information from a patient's body surface to a tumor, the internal heterogeneity information, the internal transmission information of a proton beam, and the internal motion information according to the patient's respiration into a deep learning model. The computing unit (330) may calculate a proton therapy beam angle based on an output value of the deep learning model.

The output device (340) may be a device for outputting certain information. The output device (340) may output interfaces necessary for data processing, input data, and analysis results. The output device (340) may be physically implemented in various forms such as a display or a document output device.

The interface device (350) may be a device for receiving certain commands and data from the outside. The interface device (350) may receive information and a model necessary for performing the aforementioned proton beam optimization method from a physically connected input device or an external storage device. The interface device (350) may receive a control signal for controlling the analysis apparatus (300). The interface device (350) may output analysis results of the analysis apparatus (300).

The communication device (360) may refer to a configuration that receives and transmits certain information through wired or wireless networks. The communication device (360) may receive control signals necessary for controlling the analysis apparatus (300). The communication device (360) may transmit analysis results of the analysis apparatus (300).

The aforementioned beam angle optimization method for proton therapy may be implemented as a program (or application) including an executable algorithm that can be executed by a computer.

The program may be provided by being stored in a temporary or non-transitory computer-readable medium.

The term "non-transitory computer-readable medium" refers to a medium that permanently stores data and can be read by a device, rather than a medium that stores data only for a short time such as a register, cache, or memory. Specifically, various applications or programs described above may be stored and provided in non-transitory computer-readable media such as a CD, DVD, hard disk, Blu-ray disk, USB, memory card, ROM (read-only memory), PROM (programmable read-only memory), EPROM (erasable PROM), EEPROM (electrically erasable PROM), or flash memory.

The term "temporary computer-readable medium" refers to various types of RAM, such as Static RAM (SRAM), Dynamic RAM (DRAM), Synchronous DRAM (SDRAM), Double Data Rate SDRAM (DDR SDRAM), Enhanced SDRAM (ESDRAM), Synclink DRAM (SLDRAM), and Direct Rambus RAM (DRRAM).

The embodiments and drawings attached to this specification merely illustrate some of the technical ideas included in the above-described technology, and it is obvious that all modifications and specific embodiments that can be easily derived by those skilled in the art within the technical scope of the specification and drawings are included within the scope of the present disclosure.

## Claims

1. A beam angle optimization method for proton therapy, comprising:
receiving, by an analysis apparatus, distance information from a patient's body surface to a tumor, internal heterogeneity information, internal transmission information of a proton beam, and internal motion information according to the patient's respiration;
inputting, by the analysis apparatus, the distance information from the patient's body surface to the tumor, the internal heterogeneity information, the internal transmission information of the proton beam, and the internal motion information according to the patient's respiration into a deep learning model; and
calculating, by the analysis apparatus, a proton therapy beam angle based on an output value of the deep learning model.

2. The method of claim 1, wherein the internal heterogeneity information includes information calculated based on a standard deviation value of a Hounsfield Unit (HU) obtained from a computed tomography (CT) image.

3. The method of claim 1, wherein the internal motion information according to the patient's respiration includes information calculated from deformation vector fields (DVF).

4. The method of claim 1, wherein the distance information from the patient's body surface to the tumor, the internal heterogeneity information, the internal transmission information of the proton beam, and the internal motion information according to the patient's respiration include information according to a proton beam angle.

5. The method of claim 1, wherein calculating the proton therapy beam angle includes deriving an effect corresponding to 360 degrees in the form of a peak.

6. A beam angle optimization apparatus for proton therapy, comprising:
an input device configured to receive distance information from a patient's body surface to a tumor, internal heterogeneity information, internal transmission information of a proton beam, and internal motion information according to the patient's respiration;
a computing unit configured to input the distance information from the patient's body surface to the tumor, the internal heterogeneity information, the internal transmission information of the proton beam, and the internal motion information according to the patient's respiration into a deep learning model, and to calculate a proton therapy beam angle based on an output value of the deep learning model; and
a storage device configured to store the deep learning model.

7. The apparatus of claim 6, wherein the internal heterogeneity information includes information calculated based on a standard deviation value of a Hounsfield Unit (HU) obtained from a computed tomography (CT) image.

8. The apparatus of claim 6, herein the internal motion information according to the patient's respiration includes information calculated from deformation vector fields (DVF).

9. The apparatus of claim 6 wherein the distance information from the patient's body surface to the tumor, the internal heterogeneity information, the internal transmission information of the proton beam, and the internal motion information according to the patient's respiration include information according to a proton beam angle.

10. The apparatus of claim 6, wherein calculating the proton therapy beam angle includes deriving an effect corresponding to 360 degrees in the form of a peak.
